# EUROPEAN PATENT APPLICATION

(11) **EP 4 755 485 A1**
(43) Date of publication of application: **10.06.2026**
(21) Application number: 24218305.1
(22) Date of filing: 09.12.2024
(51) Int. Cl.: B01D 11/02, A61K 31/00, A61K 36/185

(54) **CANNABIS FLOWER EXTRACTION METHOD AND EXTRACT FORMULATIONS**

(71) Applicant: Somaí Pharmaceuticals Limited, Dublin 2 D02PR63 (IE)
(72) Inventor: Sassano, Michael Anto, Carregado (PT)
(74) Representative: MacLachlan IP Limited

(57) **Abstract**

The present invention relates to a method of extracting cannabinoids and terpenes from cannabis flos comprising the steps of loading cannabis flos into a vacuum oven to produce vapour and biomass; collecting and condensing the vapour produced; separating terpenes from the condensed liquid and storing the collected terpenes for further use; removing the biomass from the oven and adding ethanol chilled to a temperature between -35 and -45°C to form a biomass ethanol mixture, percolating the ethanol solution through the mixture multiple times, keeping the solution at about - 40 to -50°C; removing solid material from the mixture to leave a crude oil tincture; filtering the tincture to remove unwanted components; evaporating ethanol from the filtered crude oil and ethanol mixture at a temperature not exceeding 100°C; decarboxylating the crude oil by thin layer heating at 120 °C to obtain a crude extract with a THC content of at least 65 w/w%; subjecting the crude extract to a plurality of wiped film distillations; blending the film wipe distillation fractions to obtain a purified extract with a THC concentration of at least 75 w/w%; and adding the collected terpenes to the purified THC extract.

## Description

The present invention relates to improvements in and relating to extraction of essential elements from cannabis plant material, in particular to a full-spectrum female flower extract retaining all positive properties of the initial crude extract wherein major cannabinoids, minor cannabinoids and terpenes, i.e., bioactive compounds, are preserved and lipids, carbohydrates, chlorophyll, and polyphenols are removed. The invention further relates to formulations of the extract with a cannabinoid delivery vehicle.

### Background to the Invention

As per the European Pharmacopoeia, cannabis flower and cannabis flos are interchangeable terms meaning dried, whole or fragmented, fully developed female inflorescence of *Cannabis sativa L.*

Cannabinoids found in *Cannabis sativa L.,* notably delta-9 tetrahydrocannabinol (THC) and cannabidiol (CBD), are the main active compounds and hold immense promise for a wide range of medical applications, including chronic pain and insomnia due to their potential therapeutic benefits, for instance, as analgesic, antioxidant, anti-cancer and neuroprotective agents. However, both THC and CBD exhibit notable challenges related to first-pass metabolism when administered orally.

Cannabinoids have a low bioavailability that is dependent on food consumed by the patient and on bile excretion. Most clinical studies have determined oral bioavailability of CBD and THC to fall between 6-10% compared to intravenous administration.

Cannabis extracts come in three major forms, depending on the extraction and purification processes. The most concentrated consists of the cannabinoid isolates. Next comes the cannabis distillate (purified cannabis oil) that lacks terpenes and sugars since the oil is stripped off during purification. These extracts undergo a purification process, resulting in amber coloured oil that ideally contains no other compounds, e.g., solvents, fats, or terpenes.

Finally, full extract cannabis oil (FECO), uses the cannabis flower but excludes the large fan leaves. FECO includes sugars, waxes, polyphenols and terpenes, along with the cannabinoids, i.e., FECO comprises all the active phytochemicals naturally present in the flower together with various undesired compounds. FECO can be produced using any solvent or non-solvent based extraction. Aside from whether high-CBD or high-THC *Cannabis sativa L.* flowers are used as the source material, FECO specifically contains THC, which can produce an intoxicatingly psychoactive "high" effect when ingested at certain levels. Other compounds found in FECO include a range of other cannabinoids including cannabinol (CBN), cannabigerol (CBG), cannabicyclol (CBL), cannabielsoin (CBE), cannabitriol (CBT) and cannabichromene (CBC), along with other cannabinoids, various terpenoids and flavonoids.

Problems with known FECO products include poor taste, smell, or both. This is because, to avoid being eaten, the cannabis plant produces resin glands (trichomes), volatile organic compounds (terpenes), and even cannabinoids (tetrahydrocannabinolic acid (THCA)). The cannabis plant is an expert in employing these chemical defences. These defence mechanism chemicals are produced from biotic or abiotic stressors. Water stress, UV stress, mechanical stress and pathogenic stress all result in the plant releasing different chemicals, most of which have little to no medicinal value.

The self-protecting compounds generated within the plant can be different acids (jasmonic acid, salicylic acid), an increase in sodium or chlorine, additional lipids to reflect harmful UV rays and overproduction of volatile organic compounds like the terpene humulene. Many of these compounds are antifeedants, with a sole purpose to ward off predators with strong bitter taste or noxious smell. When processed, these compounds are solubilised during extraction, resulting in poor taste with no scientifically accepted benefit.

When a manufacturer produces a raw extract, no matter the method of extraction, the resulting oil is composed of the acidic forms of the cannabinoids. Raw extracts must undergo decarboxylation to remove the carboxylic acid from THCA and convert the molecules to their more stable and more psychoactive counterpart, THC. To achieve decarboxylation the oil must be exposed to high temperatures for a specific residence time to break the carbon bonds and release CO₂. High heat from decarboxylation decomposes terpenes and may even lead to the creation of new compounds in the process, such as the highly reactive aldehydes and esters. These compounds will have an impact on the final taste of the decarboxylated full spectrum oil.

Medicines and other products created from unpurified full extract cannabis oil will have a range of tastes such as earthy, bitter, and grassy all of which are quite strong and generally regarded as unpleasant. Unpurified full extract cannabis oil can potentially have additional adverse effects, such as irritation, from the volatile organic compounds when exposed to sensitive tissues and membranes.

It is therefore an object of the present invention to provide a cannabis extract and extraction method which overcome the drawbacks of known solutions or at least provide suitable alternatives.

### Summary of the Invention

The present invention addresses the problem of providing a palatable full-spectrum cannabis extract whilst retaining medicinal compounds and other compounds of interest from within the dried flowers. The present invention further addresses the problem of improving oral bioavailability of CBD and THC to above 10%.

These problems are solved by utilising female cannabis flowers rather than the whole cannabis plant and by initial extraction of terpenes followed by a secondary extraction of cannabinoids with purification of the cannabinoid extract via successive distillations and possible recombination of the native terpenes and purified cannabinoid extracts obtained.

The present invention relates to a method of preparing a full-spectrum cannabis extract, a full-spectrum cannabis extract prepared by said method and a formulation containing the extract.

The invention is set out in the claims.

Accordingly, a first aspect of the present invention is a method of preparing a full-spectrum cannabis extract by extracting cannabinoids and terpenes from cannabis flos comprising the steps of:
- loading cannabis flos into a vacuum oven to produce vapour and biomass;
- collecting and condensing the vapour produced;
- separating terpenes from the condensed liquid and storing the collected terpenes for further use;
- removing the biomass from the oven and adding ethanol chilled to a temperature in the range of from about -35 and about -45°C to form a biomass ethanol mixture;
- percolating the ethanol solution through the mixture multiple times, keeping the solution at a temperature in the range of from -40 to about -50°C;
- removing solid material from the mixture with a sieve to leave a crude oil tincture;
- filtering the tincture to remove unwanted components;
- evaporating ethanol from the filtered crude oil tincture at a temperature not exceeding 100°C;
- decarboxylating the crude oil by thin layer heating at 120 °C to obtain a crude extract with a THC content of at least 65 w/w%;
- subjecting the crude extract to a first wiped film distillation to obtain a first pass distillate, said first wiped film distillation being performed at a pressure of 0.2000 to 0.8000 mbar with a product temperature in the range of from about 105 to about 115 °C, distillation surface temperature in the range of from about 140 to about 150 °C; speed up to 400rpm; flow of about 25 to about 35Hz; first step condenser temperature in the range of from about -20 to about °C and second step condenser temperature in the range of from about 45 to about 55 °C;
- subjecting the first pass distillate to a second wiped film distillation to obtain a first residue and a first film wipe distillation fraction, said second wiped film distillation being performed at a pressure of 0.1500 to 0.3500 mbar with a product temperature in the range of from about 105 to about 115 °C, distillation surface temperature in the range of from about 155 to about 165 °C; speed up to 400rpm; flow of from about 25 to about 35Hz; first step condenser temperature in the range of from about -20 to about -30 °C and second step condenser temperature in the range of from about 65 to about 75 °C;
- subjecting the first residue to at least one wiped film distillation to obtain further film distillation fractions, each further wiped film distillation being performed at the same conditions as the second wiped film distillation;
- blending the film wipe distillation fractions to obtain a purified extract with a THC concentration of at least 75 w/w%; and
- adding the collected terpenes to the purified THC extract.

The present invention does not use all of the cannabis plant, instead only dried female flowers are used. The flower contains more trichomes than other parts of the plant and trichomes are the "glands" containing the resin to be extracted. Other parts of the plant can only contribute in a marginal way and increase the risk of undesirable components like chlorophyll from leaves or bioburden contaminants from the plant stem. Cannabis flos typically has a THC concentration of at least 16 w/w%.

During each wiped film distillation, volatile components are removed without degradation of the distillation fractions, i.e., of the desired extract. The condensed volatile components obtained in the cold trap condensers are preferably discarded.

Advantageously, the removal of the terpenes prior to cannabinoid extraction according to the invention means the terpenes will not be degraded during the decarboxylation or distillation steps.

Without being bound by theory, the inventors believe that thin layer heating at 120 °C during decarboxylation avoids temperature stress and oxidation situations to the cannabinoids.

Advantageously, cryoethanol extraction and wiped film distillation ensure removal of unwanted defence mechanism chemicals that are produced from biotic or abiotic stressors. Removal of these defence mechanism chemicals is beneficial as defence mechanism chemicals result in strong tasting and bitter oils in prior art products.

The first pass distillate is substantially free of volatile substances.

The combination of cryoethanol extraction and wiped film distillations according to the invention results in purified full-spectrum extract containing beneficial compounds found within cannabis flos and selectively eliminates one or more undesired compounds selected from among waxes, chlorophyll, sugars, polyphenols and flavonoids, allowing decarboxylation of acidic cannabinoids without producing unappealing bitter tastes as well as allowing the major cannabinoid content to increase up to >85 w/w%. As cannabinoids distillate in the same range of conditions, the mix and ratio between different cannabinoids present in the plant material, is also present in the purified extract distillate.

Extracts resulting from the method according to the invention contain the full spectrum of therapeutic compounds present in cannabis flos and exclude inactive compounds that both contribute to the negative taste profile of prior art products and decrease stability. Without being bound by theory, the inventors believe that the combinations of cannabinoids and terpenes present in the products according to the invention result in additive effects compared to isolated cannabinoids, for example neuroprotective and anti-aggregatory effects against β amyloid-mediated toxicity.

In a preferred embodiment, the cannabis flos are broken down or ground prior to loading into the vacuum oven.

Additionally, or alternatively, the cannabis flos are loaded onto perforated trays, preferably wherein the trays have metal cross sections to allow more even distribution of heat through the material by maximising surface area.

The vacuum oven is preferably at a temperature in the range of from about 140°C to about 160°C, particularly at about 150°C and at a negative pressure of about 600 mbar to about 700 mbar.

Additionally, or alternatively, the cannabis flos is heated in the oven for at least 1 hour, preferably from about 1 hour to about 2 hours.

The vapours produced from the biomass on heating in the vacuum oven are preferably collected in a cold trap with a temperature in the range of from about -10°C to about -40°C set between the vacuum pump and the exhaust of the oven.

The terpenes are preferably separated from the condensed liquid using a separatory funnel where one fraction is the aqueous layer containing water and hydrosols with the second layer containing hydrophobic terpenes. The hydrosol layer is preferably chilled to a temperature in the range of from about -10°C and about -30°C and hydrophilic terpenes trapped within the frozen water subsequently separated.

In some embodiments, the method further comprises subjecting a CBD extract obtained from cannabis flos to wiped film distillations under conditions as described hereinabove for the THC extract and adding the purified CBD extract to the purified THC extract and collected terpenes. The CBD extract is preferably produced with supercritical CO₂ on decarboxylated cannabis flos with subsequent removal of coextracted waxes.

Preferably, an extract with a CBD concentration of at least 65 w/w% is subjected to a first wiped film distillation to obtain a first pass distillate, said first wiped film distillation being performed at a pressure of 0.2000 to 0.8000 mbar with a product temperature in the range of from about 105 to about 115 °C, distillation surface temperature in the range of from about 140 to about 150 °C; speed up to about 400rpm; flow in the range of from about 25 to about 35Hz; first step condenser temperature in the range of from about -20 to about -30 °C and second step condenser temperature in the range of from about 45 to about 55 °C; the first pass distillate is then subjected to a second wiped film distillation to obtain a first residue and a first film wipe distillation fraction, said second wiped film distillation being performed at a pressure in the range of from about 0.1500 to about 0.3500 mbar with a product temperature in the range of from about 105 to about 115 °C, distillation surface temperature in the range of from about 155 to about 165 °C; speed up to about 400rpm; flow in the range of from about 25 to about 35Hz; first step condenser temperature in the range of from about -20 to about-30 °C and second step condenser temperature in the range of from about 65 to about 75 °C; and the first residue is subjected to at least one wiped film distillation to obtain further film distillation fractions, each further wiped film distillation being performed at the same conditions as the second wiped film distillation. The film wipe distillation fractions are then blended to obtain a purified extract with a CBD concentration of at least 75 w/w%.

In another aspect, the invention provides an oral formulation comprising an extract prepared by the method disclosed herein and a Generally Recognised as Safe (GRAS) vehicles, preferably wherein said GRAS vehicle is glycerol monolinoleate (1-monolinolein) or a mixture of 90% glycerol monolinoleate (1-monolinolein) and 10% medium-chain triglycerides (MCT).

The oral formulation is preferably an oral solution or soft gel capsule.

Advantageously, the GRAS vehicle improves the absorption and systemic availability of THC and CBD, thus optimising therapeutic potential and mediating concerns presented by first-pass metabolism.

Advantageously, the GRAS vehicle solubilises lipophilic compounds, such as THC and CBD, improving their bioavailability, particularly for orally administered formulations. When integrated into cannabinoid formulations, the GRAS vehicle advantageously enhances the absorption of these lipophilic compounds, enabling higher concentrations in circulation, e.g., in blood and lymphatic systems. This heightened solubility leads to more efficient absorption, ultimately elevating systemic availability.

Advantageously, the GRAS vehicle aids in the even dispersion of cannabinoids, facilitating their absorption across biological barriers. The long chain unsaturated fatty acids of the GRAS vehicle improve the dispersion of THC and CBD to facilitate interaction of THC and CBD with the patient's absorptive surfaces, such as the intestinal mucosa, enhancing the rate and extent of absorption.

Advantageously, the GRAS vehicle is biocompatible and is a safe pharmaceutical excipient, minimising concerns regarding toxicity or adverse effects.

In some embodiments, the oral formulation further comprises a CBD extract, preferably a CBD extract subjected to wiped film distillations as described hereinabove for the THC extract.

The skilled person will understand that where the same feature has been referenced in different aspects of the invention, this feature comprises the same parts and operates in the same way unless otherwise stated.

### Brief Description of the drawings

Certain preferred embodiments of the present invention will now be described, by way of example only, with reference to the accompanying drawings in which:
Figure 1 is a high-level flowchart showing the key steps of a preferred method according to the invention; and
Figure 2 is a chromatographic overlay of the cannabinoid profile of cannabis flos and the cannabinoid profile of a high-THC purified extract obtained by the method shown in Figure 1 prior to addition of native terpenes.

### Detailed Description

In overview, a method for producing a full spectrum cannabis extract is provided wherein major and minor cannabinoids and terpenes are retained and lipids, carbohydrates, chlorophyll, and polyphenols are removed, the method involving extraction of THC from cannabis flos with a THC concentration of at least 16 w/w%, distillation of the crude extract with a THC concentration of at least 65 w/w%, to obtain a purified extract with a THC concentration of at least 75 w/w%, and standardisation of the purified extract.

Various embodiments of the present invention will be described in detail with reference to the drawings, where like reference numerals represent like parts and assemblies throughout the several views.

It will be appreciated that the invention should not be construed to be limited to the examples, which are now described; rather, the invention is construed to include any and all applications provided herein and all equivalent variations within the skill of the ordinary artisan.

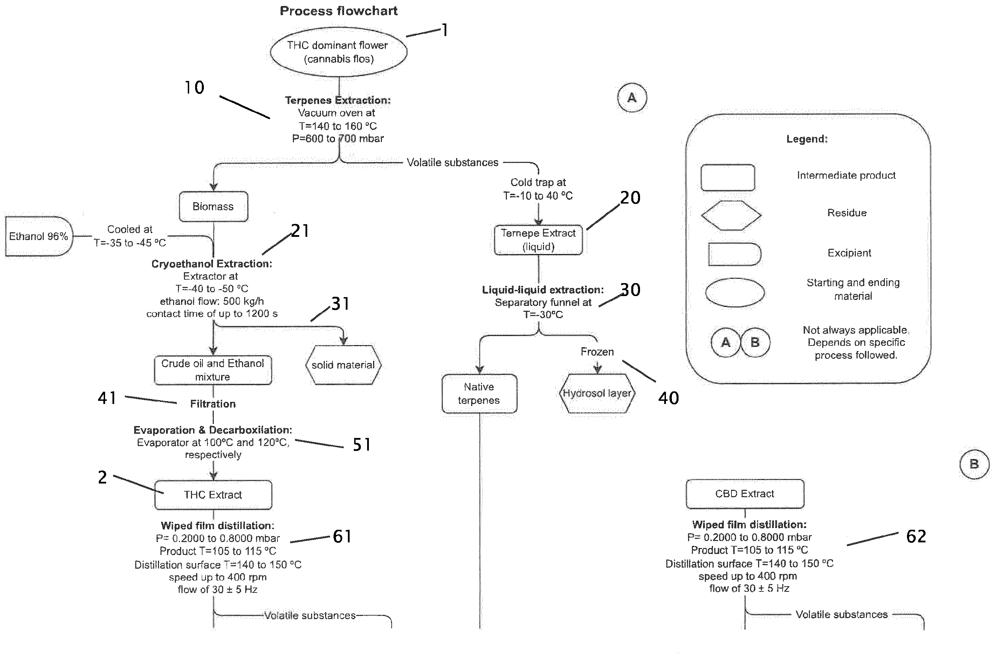

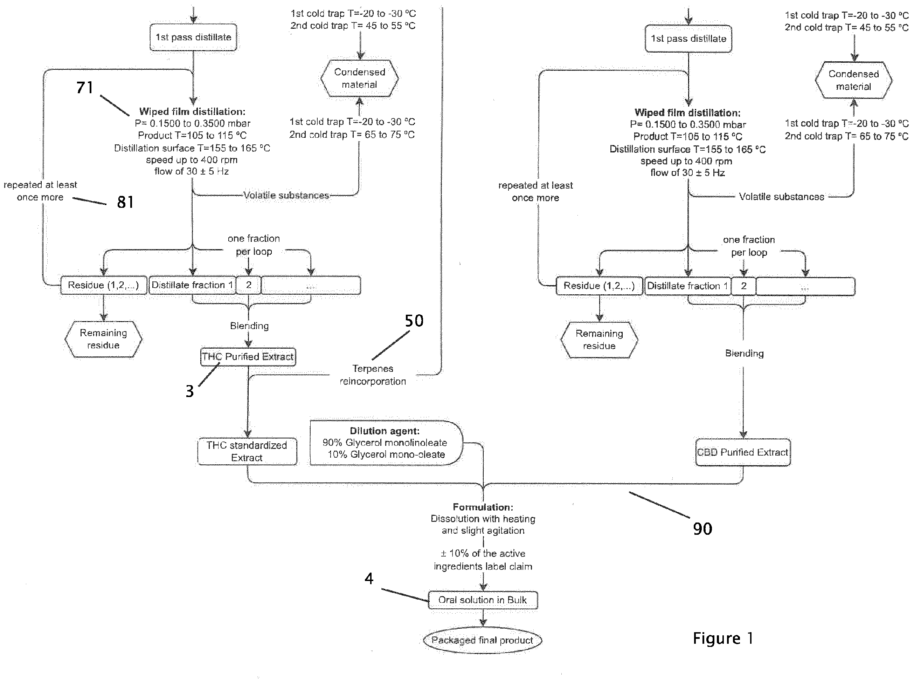

Referring to Figure 1, a flowchart of the basic steps of the method of extracting cannabinoids and terpenes from cannabis flowers according to the invention is shown.

In step 10, cannabis flos 1 with a THC content of at least 16 wt% is loaded on metal perforated trays into a vacuum oven with a temperature in the range of from about 140 to about 160 °C, e.g.,150°C, and heated for 1 to 2 hours under vacuum in the range of from about 600 to about 700 mbar to produce vapour and biomass.

Steps 20, 30 and 40 are considered to be the first extraction phase and steps 21, 22 and 31 the secondary extraction phase.

In step 20, the vapour is collected and condensed using a cold trap set between the vacuum pump and the exhaust of the oven. The cold trap is at a temperature between -10°C and - 40°C.

In step 30, hydrophobic terpenes are separated from the condensed liquid using a separatory funnel.

In step 40, the remaining aqueous layer containing water and hydrosols is chilled to -30°C and hydrophilic terpenes are separated from the frozen hydrophilic phase.

In step 21, the biomass from step 10 is removed from the vacuum oven and added to extraction equipment with ethanol (96%) chilled to -40°C as the solvent. The extraction equipment used in this step is preferably the DEVEX CryoEXS 100 manufactured by DEVEX Verfahrenstechnik GmbH. The contact time is preferably in the range of from about 10 to about 30 minutes. Typically, approximately 75 litres of ethanol is used for every 5kg of plant material, however, other ratios may be used. The ethanol solution is percolated through the biomass for about 10 to about 20 minutes to ensure all desired essential elements are extracted and subsequently drained from the biomass material to form a crude oil and ethanol mixture.

In step 31, solid material in suspension is removed from the mixture by using overheated steam pressure and a sieve to obtain a tincture;

In step 41, the crude oil and ethanol mixture is filtered through a fine mesh filter, for final removal of solids in suspension.

In step 51, ethanol is evaporated from the mixture at a temperature below 100°C using thin film distillation. The distillation equipment used in this step is preferably the V DEVEX CryoEXS 100 manufactured by DEVEX Verfahrenstechnik GmbH.

The crude oil remaining after the evaporation is decarboxylated by thin layer heating at 120 °C to obtain crude extract 2 with a THC content of at least 65 w/w%. The duration of this decarboxylating process is determined by sampling and determination of weight loss using a thermos-scale.

In step 61, the crude extract is subjected to a first wiped film distillation to remove volatile components, said first film wipe distillation being performed at a pressure of 0.2000 to 0.8000 mbar with a product temperature of 105 to 115 °C, distillation surface temperature of 140 to 150 °C; first step condenser temperature of -20 to -30 °C and second step condenser temperature of 45 to 55 °C. The distillation equipment used is the VTA VK 100-10 with degasser, manufactured by VTA Verfahrenstechnik GmbH.

Wiped film distillation is used to separate compounds based on their boiling point. All decarboxylated cannabinoids have relatively similar boiling points ranging between 105°C and 180°C. Utilising a vacuum depth of -2Mbar, or lower, distillation temperatures are kept below 175°C with a residence time in the range of from about 2 seconds to about 10 seconds. As the decarboxylated extract heats, it enters a central "wiper" area that travels in a clockwise motion, effectively pushing the melted extract against the heated walls downwards, while the vapour wants to travel upwards towards a condenser. This motion creates theoretical plates, or potential areas that the vapour can condense, which forces compounds that may have co-distilled at abnormal temperatures to condense and go to the residue waste.

In step 71, the residue from step 61, i.e., the first pass distillate substantially free of volatile substances, is subjected to a second wiped film distillation to obtain a second residue, said first film wipe distillation being performed at a pressure of 0.1500 to 0.3500 mbarwith a product temperature of 105 to 115 °C, distillation surface temperature of 155 to 165 °C; first step condenser temperature of -20 to -30 °C and second step condenser temperature of 65 to 75 °C.

In step 81, the second residue is subjected to a further two to eight, preferably a maximum of four, film wipe distillations to obtain purified extract 3 with a THC concentration of at least 75 w/w%, each further film wipe distillation being performed at the same conditions as the second wiped film distillation.

To further guarantee purity, the primary condenser is set to 75°C. Vapour that reaches the primary fraction condenser will only condense if it contains cannabinoids. Volatile organic compounds that may have co-distilled will travel beyond the condenser and instead collect at a secondary cold trap set to -90°C. This results in a purer full-spectrum extract that contains all of the cannabinoids that began in the flowers. The distillation effectively separates the cannabinoids from the terpenes, polyphenols and lipids. Residual solvents in the oil, such as ethanol, boil and collect within cold traps at a temperature of -90°C. Lipids, chlorophyll, and polyphenols will not distil and instead travel to the waste of the process. In other words, without being bound by theory, the inventors believe that the vast majority of any undesirable, foul tasting, compounds are left in the residue waste because their boiling points are either much lower, or higher, than the cannabinoids.

In step 50, the hydrophilic terpenes from step 40 are combined with the hydrophobic terpenes from step 30 and the resulting terpene mixture is added to the purified extract obtained in step 81.

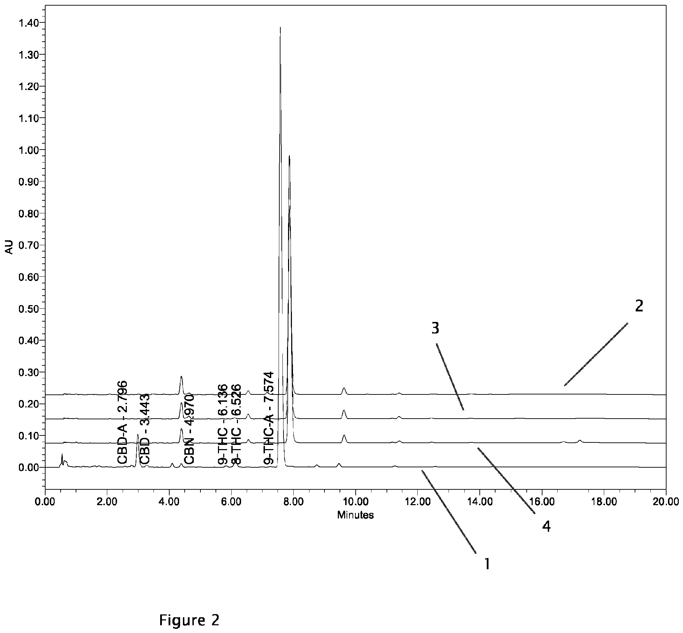

The final purified FECO oil 4 produced has a total active cannabinoid concentration (major and minor cannabinoids) of 95% with the remaining 5% being impurities such as for example waxes and chlorophyll. The major cannabinoid in oil 4, e.g., THC or CBD, has a concentration of more than 85%. The additive effects of the cannabinoids remain because the exact ratio of different cannabinoids present in the flower is retained in the purified extract, there is no relative concentration variation. Please see Figure 2 in this regard which shows chromatograms obtained for each of cannabis flos 1, crude extract 2, purified extract 3 and final purified product, i.e., oral solution 4.

Terpenes are reintroduced to the purified extract after distillation to create herbal preparations, avoiding heat related degradation and production of potentially negative byproducts. The issue of terpene loss due to extraction and terpene destruction during decarboxylation of raw extract is this avoided in the method according to the invention.

Optionally, the method further comprises step 62 in which a CBD extract is subjected to wiped film distillations under the same conditions as described above for the THC extract and step 90 in which the purified CBD extract is added to the purified THC extract and collected terpenes from step 50.

### Herbal preparation

The cannabis flower used in the following example was obtained from *Cannabis sativa (L.,* 1753) as follows:
Propagation - approximate duration 4 weeks

Asexual reproduction (by clones or cuttings) of cannabis plants from mother plants in good hydration, green leaves, and good vigour; free from pests and diseases. This stage lasts.
- Preparing trays with substrate
- Selection and preparation of cuttings - cuttings obtained from young lateral branches and at least two nodes long
- Hormone application and cutting planting: Plants without mechanical damage and spray irrigation. Temperature: 21-28 °C and %RH: 70- 90%.

### Vegetative Stage - Approximate duration 8 weeks

The vegetative phase is a period of the growing cycle that takes place after propagation and before flowering. During this stage, the plant develops its green tissues to be able to perform photosynthesis. At this stage, the plants are planted in the greenhouses directly in the soil.
- Pinch of plants: when the main stem has 4 to 5 internodes.
- Flowering induction: when the plant has reached a height greater than 80 cm.

### Flowering Stage - can last from 6 to 10 weeks

At this stage, secondary metabolites accumulate in the resinous glands (glandular trichomes) of female inflorescence. The harvested part is the female inflorescence (flower or bud). Verification of lightning system (12h light + 12h daily darkness). Initiation of monitoring plan for identification and elimination of possible males and/or hermaphrodites.
- Harvest Scheduling: at least 52 days from induction to flowering; microscopic observation of trichomes in the flower and should be approx. 50% amber and 50% crystalline.

### Post-harvest

Here plant material is prepared for the extraction process (lasts approx. 1 week). During the post-harvest process, the material only goes to a water loss process by an airflow drying tunnel (T: 10 - 70 °C; %RH: from 10% to 85%). Optionally, the plant material may be treated with gamma irradiation to lower bioburden levels. However, the plant material typically does not suffer any additional stabilisation process such UV, freezing, etc.
- Separation of the flower from the stem and weighing: Removal of leaves and stems. Drying process: Between 28 °C and 35 °C, in darkness. Completion of drying when the material has a 12% RH maximum.

Grinding and homogenisation: Granulometry of approx. 1.4 mm.

### Example 1 - Method of manufacture of Cannabis sativa L. herbal preparations containing 20mg THC per ml

The manufacturing process comprises four main steps:
1) Terpene extraction
2) Cannabinoid extraction
3) Distillation/Purification
4) Reincorporation of previously extracted terpenes and standardisation of formulation Terpene Extraction

In this first step, the flower is subjected to a temperature increase from room temperature (approximately 25 °C) of up to 150°C under vacuum conditions of up to - 700 millibar. The volatile components, i.e., terpenes and water vapour, are collected in a cold trap at -25°C. The liquid obtained in the cold trap is then frozen at a temperature in the range of from about - 10 °C to about - 30 °C and the solid frozen aqueous phase is separated from the liquid terpene-containing phase.

### Cannabinoid Extraction

In this step, 80.00 kg dried cannabis flower with a THC content >16% w/w is extracted with 1200 L ethanol (96%) at an input temperature of about -40 C, ethanol flow of about 500 kg/h and contact time of up to about 1200 seconds and up to about 1200 seconds for extraction/percolation phases.

The liquid obtained through the extraction/ percolation process goes to the evaporation and decarboxylation phases. The temperature to evaporate the extraction solvent in the dedicated equipment should not exceed 100°C. In the decarboxylation phase, this equipment should operate at 120°C for 30 minutes.

### In-Process Controls (IPC) to produce the Extract THC >65%

| **Step of IPC** | **Tests** | **Specifications if applicable** |
|---|---|---|
| Biomass after steam *(means discharged Cannabis flower after extraction phase)* | LOD | Monitorisation |
| | Cannabinoids assay | |
| Tincture (Micelle) *(liquid between extraction and evaporation phases)* | | |
| Decarboxylated oil *(in the course of time* - *until end point)* | LOD | *≤* 2,5% |
| Decarboxylated oil *(in the course of time* - *until end point)* | THC | Monitorisation |

12.500 g (±20%) intermediate extract with THC content of >65% is obtained.

### Distillation/Purification

In this step, the process starts with a desolvation phase in which the >65% intermediate THC extract is distilled at a flow of 30 ± 5 Hz and a maximum speed of about 400 rpm to remove residual solvent and any remaining terpenes.

After the desolvation phase, a plurality of wiped film distillations, preferably from 3 to 8 wiped film distillations, are carried out at a flow of 30 ± 5 Hz and a maximum speed of 400 rpm. 10.000 g purified intermediate extract with a THC content of >75% is obtained.

### In-Process Controls (IPC) to produce the Purified Extract THC >75%

| **Step of IPC** | **Tests** | **Specifications, if applicable** |
|---|---|---|
| **Desolvation & Distillation** | | |
| Desolvation: solvent residue | Cannabinoids assay | Monitorisation |
| Distillation; Semi-finished Crude 2nd Waste | | |

| **Homogenisation** | | |
|---|---|---|
| Purified Extract THC >75% | Visual inspection | Yellow to amber, solid at room temperature |
| Purified extract (centre top) | Cannabinoids assay | Monitorisation |
| Purified extract (centre side) | | |
| Purified extract (centre bottom) | | |
| Purified extract (side bottom) | | |

| **Filling/Weighing/Labelling** | | |
|---|---|---|
| Purified Extract THC >75% | Weight | >=0% ≤ 1% |
| | Closure | Check |
| | Label placed correctly | Comply |
| | Product desiqnation | Comply |
| | Expiry date | |
| | Batch number | |

### Standardisation

The next step involves pre-heating and incorporation of the previously obtained plant native terpenes followed by homogenisation steps as well as the standardisation of the purified extract the desired THC concentration, for example, THC soft extract 20 mg/ml, by addition of a mixture of 90% Glycerol monolinoleate (1-Monolinolein) and 10% MCT.

The standard batch size for the THC soft extract 20 mg/mL is 30 litres.

Qualitative and quantitative composition of the batch formula is described in the following table:

### THC soft extract 20 mg/mL batch formula

| **Component** | **Quantity*** | **Method** |
|---|---|---|
| *Intermediate of standardised extract* | | |
| Purified Extract THC 75w/w% | 800g | As described herein |

| *Dilution agent* | | |
|---|---|---|
| Glycerol monolinoleate oil | 24.930kg | European Pharmacopoeia |
| MCT | 2.770Kg | European Pharmacopoeia |

| | | |
|---|---|---|
| * Calculated based on a solution density of 0.95 g/ml | | |

If the purified extract has a higher THC concentration, then less is used. For example, for an 80w/w% concentration, 750g is used instead of 800g and so on.

### Formulation

The purified THC extract is weighed in the necessary quantity to obtain the final desired (standardised) concentration of the major cannabinoid, e.g., THC 10 mg/ml.

The process starts with the individual dispensing of the oily excipients, which are weighed, placed in reactor and subjected to slight agitation. The purified THC extract obtained above is then heated, weighed and subsequently added to the reactor containing the oily excipients for dissolution process to take place.

The obtained solution is then transferred to a buffer reactor in which it remains until the filling phase where the obtained solution is filled into suitable containers and labelled.

An inert gas such as nitrogen is applied at a flow rate of 12 L/hr to the headspace of each filled bottle prior to capping.

During the process, the following In-Process Controls (IPC) were found to be appropriate:

### In-Process Controls (IPC) to produce THC soft extract 20 mg/mL

| **Step of IPC** | **Tests** | **Specifications, if applicable** |
|---|---|---|
| Solution inside reactor *(at the end of dissolution)* | Aspect | Colourless to yellowish liquid |
| | Cannabinoids assay | Monitorisation |
| | Density | |

In order to evaluate the performance of the purification process, the cannabinoids present in each distillate fraction were identified and quantified by high performance liquid chromatography (HPLC) and photodiode array (PDA) detection using the Waters Acquity Arc system, a quaternary-based liquid chromatography system, based on the German Pharmacopeia (DAB) Cannabis flower monograph for MA-006 and Cannabis extract monograph for MA-008. These results are shown in Figure 2.

The terpenes present were identified and quantified by Gas Chromatography using standards.

### Example 2- Method of manufacture of Cannabis sativa L. herbal preparations containing 10mg THC + 10 mg CBD per ml and native terpenes

The only change to the manufacturing process described in connection with Example 1 is in step 4.

The standard batch size for the THC 10 mg/ml + CBD 10 mg/ml is 30 liters.

Qualitative and quantitative composition of the batch formula is described in the following table:

### THC+CBD soft extract 10+10 mg/mL batch formula

| **Component** | **Quantity** | **Reference to Method** |
|---|---|---|
| *Intermediate of standardised extract* | | |
| Purified Extract THC 75w/w% | 400g | As described herein |
| Purified Extract CBD 75w/w% | 400g | As described herein |

| *Dilution agent* | | |
|---|---|---|
| Glycerol monolinoleate oil | 27.700 kg | European Pharmacopoeia |

If the purified extract has a higher concentration, then less is used.

The Purified Extract THC >75% is weighed in the necessary quantity to obtain the final desired (standardised) concentration of the major cannabinoid, i.e., THC 10 mg/ml. The Purified Extract CBD >75% is weighed in the necessary quantity to obtain the final desired (standardised) concentration of the major cannabinoid, i.e., CBD 10 mg/ml.

The process starts with the dispensing of the oily excipient, which is weighed and placed in the reactor subjected to slight agitation. The purified THC and CBD extracts are heated for melting, weighed individually and subsequently added to the reactor containing the oily excipients and the dissolution process take place.

It is to be understood that the invention is not limited to the specific details described herein which are given by way of example only and that various modifications and additions are possible without departing from the scope of the invention as defined in the appended claims.

## Claims

1. A method of extracting cannabinoids and terpenes from cannabis flos comprising the steps of:
loading cannabis flos into a vacuum oven to produce vapour and biomass;
collecting and condensing the vapour produced;
separating terpenes from the condensed liquid and storing the collected terpenes for further use;
removing the biomass from the oven and adding ethanol chilled to a temperature in the range of from about -35 to about -45°C to form a biomass ethanol mixture, percolating the ethanol solution through the mixture multiple times, keeping the solution at a temperature in the range of from about -40 to about -50°C;
removing solid material from the mixture to leave a crude oil tincture;
filtering the tincture to remove unwanted components;
evaporating ethanol from the filtered crude oil and ethanol mixture at a temperature not exceeding 100°C;
decarboxylating the crude oil by thin layer heating at about 120 °C to obtain a crude extract with a THC content of at least 65 w/w%;
subjecting the crude extract to a first wiped film distillation to obtain a first pass distillate, said first wiped film distillation being performed at a pressure in the range of from about 0.2000 to about 0.8000 mbar with a product temperature of from about 105 to about 115 °C, distillation surface temperature in the range of from about 140 to about 150 °C; speed up to about 400rpm; flow in the range of from about 25 to about 35Hz; first step condenser temperature in the range of from about -20 to about-30 °C and second step condenser temperature in the range of from 45 to about 55 °C;
subjecting the first pass distillate to a second wiped film distillation to obtain a first residue and a first film wipe distillation fraction, said second wiped film distillation being performed at a pressure in the range of from about 0.1500 to about 0.3500 mbar with a product temperature in the range of from about 105 to about 115 °C, distillation surface temperature of about 155 to about 165 °C; speed up to about 400rpm; flow in the range of from about 25 to about 35Hz; first step condenser temperature in the range of from about -20 to about -30 °C and second step condenser temperature in the range of from about 65 to about 75 °C;
subjecting the first residue to at least one wiped film distillation to obtain further film distillation fractions, each further wiped film distillation being performed at the same conditions as the second wiped film distillation;
blending the film wipe distillation fractions to obtain a purified extract with a THC concentration of at least 75 w/w%; and
adding the collected terpenes to the purified THC extract.

2. The method as claimed in claim 1, wherein the method further comprises subjecting a CBD extract to a first wiped film distillations.to obtain a first pass distillate, said first wiped film distillation being performed at a pressure in the range of from about 0.2000 to about 0.8000 mbar with a product temperature in the range of from about 105 to about 115 °C, distillation surface temperature in the range of from about 140 to about 150 °C; speed up to about 400rpm; flow in the range of from about 25 to about 35Hz; first step condenser temperature in the range of from about -20 to about -30 °C and second step condenser temperature in the range of from about 45 to about 55 °C; subjecting the first pass distillate to a second wiped film distillation to obtain a first residue and a first film wipe distillation fraction, said second wiped film distillation being performed at a pressure of about 0.1500 to about 0.3500 mbar with a product temperature in the range of from about 105 to about 115 °C, distillation surface temperature in the range of from about 155 to about 165 °C; speed up to about 400rpm; flow in the range of from about 25 to about 35Hz; first step condenser temperature in the range of from about -20 to about -30 °C and second step condenser temperature in the range of from about 65 to about 75 °C; and adding the purified CBD extract to the purified THC extract and collected terpenes.

3. The method as claimed in claim 1 or claim 2, wherein the terpenes are separated from the condensed liquid using a separatory funnel at about -30°C.

4. The method as claimed in any one of claims 1 to 3, wherein the vacuum oven is at a temperature in the range of from about 140°C to about 160 °C and at a negative pressure of about 600 mbar to about 700 mbar.

5. The method as claimed in any one of the preceding claims, wherein the cannabis flos is heated in the oven for at least 1 hour.

6. The method as claimed in any one of the preceding claims, wherein the vapours produced from the biomass on heating in the vacuum oven are collected in a cold trap with temperature in the range of from about -10°C to about -40°C set between the vacuum pump and the exhaust of the oven.

7. An oral formulation comprising an extract obtained by the method of any one of claims 1 to 6 and a GRAS vehicle.

8. The oral formulation of claim 7, wherein the GRAS vehicle comprises glycerol monolinoleate.

9. The oral formulation of claim 8, wherein the GRAS vehicle comprises glycerol monolinoleate and medium-chain trigylcerides in a 9:1 ratio.
